# EUROPEAN PATENT APPLICATION

(11) **EP 4 385 972 A1**
(43) Date of publication of application: **19.06.2024**
(21) Application number: 22855841.7
(22) Date of filing: 04.08.2022
(51) Int. Cl.: C07C 2/02, C07C 2/34, C07C 5/13, C07C 11/02, C10G 45/02, C10G 50/02, C10N 30/06, C10M 107/02, C07B 61/00, B01J 23/44, B01J 31/22

(54) **METHOD FOR PRODUCING ALPHA-OLEFIN OLIGOMER COMPOSITION**

(30) Priority: 11.08.2021 JP 2021131433
(71) Applicant: Idemitsu Kosan Co.,Ltd., Tokyo 100-8321 (JP)
(72) Inventor: OBA, Kouta, Tokyo 100-8321 (JP); KAGAMI, Narinobu, Tokyo 100-8321 (JP); ISAJI, Mio, Tokyo 100-8321 (JP); UEDA, Naoyuki, Tokyo 100-8321 (JP); SAKAGUCHI, Takahiro, Tokyo 100-8321 (JP); KATAYAMA, Kiyokazu, Tokyo 100-8321 (JP); YOKOTA, Kiyohiko, Tokyo 100-8321 (JP); KOMURO, Yuji, Tokyo 100-8321 (JP); SAMEJIMA, Kanako, Tokyo 100-8321 (JP); TOKUNAGA, Michika, Tokyo 100-8321 (JP)
(74) Representative: Gille Hrabal Partnerschaftsgesellschaft mbB Patentanwälte
(86) International application number: PCT/JP2022/029968
(87) International publication number: WO 2023/017779

(57) **Abstract**

A method for producing an α-olefin oligomer composition includes: an oligomerization step A1 of oligomerizing an α-olefin with a metallocene catalyst to obtain an oligomer (a); a distillation separation step A2 of distilling and separating the oligomer (a) to obtain a high molecular weight fraction (a1) and a low molecular weight fraction (a2); an oligomerization step B 1 of oligomerizing an α-olefin oligomer containing the low molecular weight fraction (a2) with an acid catalyst to obtain an oligomer (b); and a mixing step C of mixing a portion of the oligomer (a) or a hydrogenated product thereof and a portion or a whole of the oligomer (b), an isomer thereof, or a hydrogenated isomer thereof.

## Description

### Technical Field

The present invention relates to a method for producing an α-olefin oligomer composition.

### Background Art

In recent years, lubricating oils are required to have high long-drain capability from the viewpoint of resource saving as well as environmental protection. In order for a lubricating oil to have high long-drain capability, the lubricating oil needs to be stable to heat and oxidation reaction.

Various synthetic lubricating oils have been developed as such lubricating oils having excellent stability. For example, a poly(α-olefin), a polybutene, an alkylbenzene, a polyol ester, a dibasic acid ester, a polyoxyalkylene glycol, a polyoxyalkylene glycol ester, a polyoxyalkylene glycol ether, or a silicone is used as a synthetic lubricant base oil.

Among them, a poly(α-olefin), which has high chemical stability and an excellent viscosity index, is widely used. A poly(α-olefin) having an intended viscosity is produced by adjusting an α-olefin as a starting material and the polymerization degree, and is practically used. Further, a production process has been investigated to efficiently obtain a poly(α-olefin) having a controlled molecular weight and structure.

For example, PTL 1 discloses a method for producing an α-olefin oligomer composition including a step of reacting an α-olefin in the presence of hydrogen with a specific catalyst and co-catalyst in order to reduce the catalytic amount and obtain an oligomer having a specific polymerization degree with a high selectivity. PTL 2 discloses a method for obtaining a component of a lubricating oil composition, which is excellent in oxidation stability, heat stability, and low-temperature stability. The method includes dimerizing an α-olefin in the presence of a metallocene complex catalyst to produce a vinylidene olefin, further dimerizing the vinylidene olefin in the presence of an acid catalyst, and then hydrogenating the resulting dimer to produce a saturated aliphatic hydrocarbon compound.

PTL 3 discloses a method for obtaining a low-viscosity oil including 9-methyl-11,13-dioctyltricosane. The method involves oligomerizing 1-decene in the presence of hydrogen, a metallocene catalyst, and an activator compound, catalytically hydrogenating the oligomerized product in the presence of hydrogen and a hydrogenation catalyst, and isolating the resulting tetramer fraction by distillation under reduced pressure.

### Citation List

### Patent Literature

PTL 1: WO2010/053022 A
PTL 2: JP 2006-342149 A
PTL 3: JP 2018-519393 A

### Summary of Invention

### Technical Problem

Since an oligomer having a specific polymerization degree is selectively obtained with a metallocene catalyst like PTL 1, the methods are an excellent method but depend on the application of the oligomer. However, when an oligomer having a relatively high polymerization degree that is required in recent years is particularly obtained as a lubricating oil component, the distribution of polymerization degree increases, and the selectivity is deteriorated, resulting in a reduction in yield and a reduction in performance.

Therefore, a method for obtaining an α-olefin oligomer having excellent selectivity as well as excellent performance during use as a lubricating oil regardless of the polymerization degree is required.

An object of the present invention is to provide a method for producing an α-olefin oligomer composition that can selectively obtain an α-olefin oligomer having an intended polymerization degree and obtain an α-olefin oligomer composition having excellent performance during use as a lubricating oil.

### Solution to Problem

The present inventors have intensively studied to achieve the object, and as a result, found that the object can be achieved by a production method including an oligomerization step with a metallocene catalyst, a distillation separation step, and an oligomerization step using a low molecular weight fraction obtained in the distillation separation step in combination, and further a mixing step of mixing the oligomers and the like.

Thus, the present invention relates to the following <1> to <16>.
<1> A method for producing an α-olefin oligomer composition including: an oligomerization step A1 of oligomerizing an α-olefin with a metallocene catalyst to obtain an oligomer (a); a distillation separation step A2 of distilling and separating the oligomer (a) to obtain a high molecular weight fraction (a1) and a low molecular weight fraction (a2); an oligomerization step B 1 of oligomerizing an α-olefin oligomer containing the low molecular weight fraction (a2) with an acid catalyst to obtain an oligomer (b); and a mixing step C of mixing a portion of the oligomer (a) or a hydrogenated product thereof and a portion or a whole of the oligomer (b), an isomer thereof, or a hydrogenated isomer thereof.
<2> The method for producing an α-olefin oligomer composition according to <1>, further including a distillation separation step D of separating a mixture obtained in the mixing step C into a plurality of fractions by distillation.
<3> The method for producing an α-olefin oligomer composition according to <1> or <2>, further including a hydrogenation step A3 of hydrogenating the high molecular weight fraction (a1) to obtain a hydrogenated oligomer (a11), wherein the hydrogenated oligomer (a11) is subjected to the mixing step C.
<4> The method for producing an α-olefin oligomer composition according to any one of <1> to <3>, wherein the α-olefin in the oligomerization step A1 is 1-decene.
<5> The method for producing an α-olefin oligomer composition according to any one of <1> to <4>, wherein the high molecular weight fraction (a1) is a fraction with more than 20 carbon atoms, and the low molecular weight fraction (a2) is a fraction with 20 or less carbon atoms.
<6> The method for producing an α-olefin oligomer composition according to any one of <1> to <5>, wherein the metallocene catalyst contains a transition metal compound represented by a general formula (1) described below: 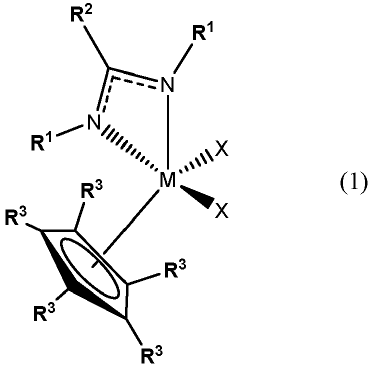 wherein M represents zirconium or hafnium, R¹'s represent an alkyl group having 1 to 10 carbon atoms or a silyl group substituted by an alkyl group having 1 to 10 carbon atoms, R² represents a phenyl group or a phenyl group substituted by an alkyl group having 1 to 10 carbon atoms, R³'s represent a hydrogen atom or a methyl group, and X's represent a halogen atom or an alkyl group having 1 to 10 carbon atoms, provided that two R¹'s may be the same as or different from each other and five R³'s may be the same as or different from each other.
<7> The method for producing an α-olefin oligomer composition according to any one of <1> to <5>, wherein the metallocene catalyst contains a transition metal compound represented by a general formula (2) described below:

   {C₅H₄(AR⁴R⁵R⁶)}₂ZrCl₂ (2)

   wherein R⁴'s, R⁵'s, and R⁶'s each independently represent a hydrocarbon group having 1 to 10 carbon atoms, two or more thereof may bind to each other to form a ring, and A represents a Group 14 element in the periodic table.
<8> The method for producing an α-olefin oligomer composition according to any one of <1> to <5> or <7>, wherein the metallocene catalyst contains a transition metal compound represented by a formula (3) described below:

   {C₅H₄(CMe₃)}₂ZrCl₂ (3).
<9> The method for producing an α-olefin oligomer composition according to any one of <1> to <8>, wherein the metallocene catalyst further contains an organoaluminum oxo compound or an organoaluminum compound and an organoboron compound.
<10> The method for producing an α-olefin oligomer composition according to any one of <1> to <9>, further including an isomerization step B2 of isomerizing the oligomer (b) to obtain an isomer; and a hydrogenation step B3 of hydrogenating the isomer to obtain a hydrogenated isomer, wherein the hydrogenated isomer is subjected to the mixing step C.
<11> The method for producing an α-olefin oligomer composition according to <10>, further including a distillation separation step B4 of distilling and separating the hydrogenated isomer obtained in the hydrogenation step B3 to obtain a high molecular weight fraction (b11) and a low molecular weight fraction (b21), wherein the high molecular weight fraction (b11) is subjected to the mixing step C.
<12> The method for producing an α-olefin oligomer composition according to <11>, wherein the high molecular weight fraction (b11) is a fraction with more than 20 carbon atoms, and the low molecular weight fraction (b21) is a fraction with 20 or less carbon atoms.
<13> The method for producing an α-olefin oligomer composition according to any one of <1> to <12>, wherein the acid catalyst for use in the oligomerization step B1 is a Friedel-Crafts catalyst.
<14> The method for producing an α-olefin oligomer composition according to any one of <10> to <13>, wherein the isomerization step B2 is isomerization in the presence of the Friedel-Crafts catalyst.
<15> The method for producing an α-olefin oligomer composition according to <13> or <14>, wherein the Friedel-Crafts catalyst contains an organoaluminum compound.
<16> The method for producing an α-olefin oligomer composition according to any one of <2> to <15>, wherein the distillation separation step D is short path distillation.

### Advantageous Effects of Invention

The present invention can provide a method for producing an α-olefin oligomer composition that can selectively obtain an α-olefin oligomer having an intended polymerization degree and obtain an α-olefin oligomer composition having excellent performance during use as a lubricating oil.

### Description of Embodiments

The present invention is a method for producing an α-olefin oligomer composition including: an oligomerization step A1 of oligomerizing an α-olefin with a metallocene catalyst to obtain an oligomer (a); a distillation separation step A2 of distilling and separating the oligomer (a) to obtain a high molecular weight fraction (a1) and a low molecular weight fraction (a2); an oligomerization step B1 of oligomerizing an α-olefin oligomer containing the low molecular weight fraction (a2) with an acid catalyst to obtain an oligomer (b); and a mixing step C of mixing a portion of the oligomer (a) or a hydrogenated product thereof and a portion or the whole of the oligomer (b), an isomer thereof, or a hydrogenated isomer thereof.

Hereinafter, each of the steps of the present invention and an optional step will be described in detail.

### [Oligomerization Step A1]

The oligomerization step A1 is an oligomerization step of oligomerizing an α-olefin with a metallocene catalyst to obtain an oligomer (a).

### <Metallocene Catalyst>

As the metallocene catalyst used in the oligomerization step A1, a catalyst containing (i) a transition metal compound (metallocene complex) that has a ligand having a conjugated five-membered carbon ring and contains zirconium or hafnium, and (ii) an aluminum compound is preferably used.

From the viewpoint of catalytic activity, the transition metal compound (metallocene complex) that has the ligand having the conjugated five-membered carbon ring and contains zirconium or hafnium (i) that constitutes the catalyst preferably includes at least one selected from the group consisting of a transition metal compound represented by the following general formula (1) and a transition metal compound represented by the following general formula (2), and more preferably includes the transition metal compound represented by the general formula (2). At least one selected from the group consisting of the transition metal compound represented by the following general formula (1) and the transition metal compound represented by the following general formula (2) is preferred, and the transition metal compound represented by the general formula (2) is more preferred. Of the transition metal compound represented by the general formula (2), a transition metal compound represented by the following formula (3) is more preferred. The transition metal compound represented by the formula (3) is bis(t-butylcyclopentadienyl)zirconium dichloride.

A solution in which the transition metal compound (metallocene complex) is dissolved in a solvent is preferably used. Examples of the used solvent include aromatic hydrocarbons such as benzene, toluene, xylene, and ethylbenzene. Toluene is preferred.

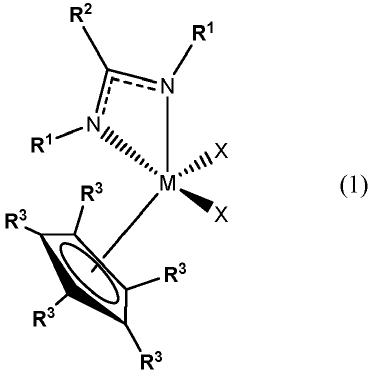

{C₅H₄(AR⁴R⁵R⁶)}₂ZrCl₂ (2)

{C₅H₄(CMe₃)}₂ZrCl₂ (3)

In the formula (1), M represents zirconium or hafnium, R¹'s represent an alkyl group having 1 to 10 carbon atoms or a silyl group substituted by an alkyl group having 1 to 10 carbon atoms, R² represents a phenyl group or a phenyl group substituted by an alkyl group having 1 to 10 carbon atoms, R³'s represent a hydrogen atom or a methyl group, and X's represent a halogen atom or an alkyl group having 1 to 10 carbon atoms, provided that two R¹'s may be the same as or different from each other and five R³'s may be the same as or different from each other.

In the formula (2), R⁴'s, R⁵'s, and R⁶'s each independently represent a hydrocarbon group having 1 to 10 carbon atoms, two or more thereof may bind to each other to form a ring, and A represents a Group 14 element in the periodic table.

In the formula (3), Me represents a methyl group (CH₃).

Examples of the alkyl group having 1 to 10 carbon atoms as R¹'s and X's in the transition metal compound represented by the general formula (1) include a methyl group, an ethyl group, a n-propyl group, an isopropyl group, a n-butyl group, an isobutyl group, a t-butyl group, various types of pentyl groups, a hexyl group, a heptyl group, an octyl group, a nonyl group, and a decyl group.

Examples of the silyl group substituted by the alkyl group having 1 to 10 carbon atoms as R¹'s include a trialkyl group-substituted silyl group that is substituted by the above-described alkyl group.

Specific examples thereof include a trimethylsilyl group, a methyldiphenylsilyl group, a dimethylphenylsilyl group, a triphenylsilyl group, and a triethylsilyl group. A trimethylsilyl group is preferred.

Examples of the phenyl group substituted by the alkyl group having 1 to 10 carbon atoms as R² include a mono- to penta-substituted phenyl group that is substituted by the above-described alkyl group.

Specific examples thereof include a 4-methylphenyl group, a 1,3,5-trimethylphenyl group, a 2,5-dimethylphenyl group, a 2,5-diisopropylphenyl group, and a 1,3,5-triisopropylphenyl group.

Examples of the halogen atom as X's include a fluorine atom, a chlorine atom, a bromine atom, and an iodine atom.

Specific examples of the transition metal compound represented by the general formula (1) wherein M is hafnium include pentamethylcyclopentadienyl[N,N'-bis(trimethylsilyl)benzamidinate]hafnium dichloride, pentamethylcyclopentadienyl[N,N'-bis(trimethylsilyl)benzamidinate]hafnium dibromide, cyclopentadienyl[N,N'-bis(trimethylsilyl)benzamidinate]hafnium dichloride, cyclopentadienyl[N,N'-bis(trimethylsilyl)benzamidinate]hafnium dibromide, pentamethylcyclopentadienyl(dimethylbenzamidinate)hafnium dichloride, pentamethylcyclopentadienyl(dimethylbenzamidinate)hafnium dibromide, cyclopentadienyl(dimethylbenzamidinate)hafnium dichloride, and cyclopentadienyl(dimethylbenzamidinate)hafnium dibromide. Pentamethylcyclopentadienyl[N,N'-bis(trimethylsilyl)benzamidinate]hafnium dichloride and cyclopentadienyl[N,N'-bis(trimethylsilyl)benzamidinate]hafnium dichloride are preferred.

Specific examples of the transition metal compound represented by the general formula (1) wherein M is zirconium include pentamethylcyclopentadienyl[N,N'-bis(trimethylsilyl)benzamidinate]zirconium dichloride, pentamethylcyclopentadienyl[N,N'-bis(trimethylsilyl)benzamidinate]zirconium dibromide, pentamethylcyclopentadienyl(dimethylbenzamidinate)zirconium dichloride, pentamethylcyclopentadienyl(dimethylbenzamidinate)zirconium dibromide, cyclopentadienyl[N,N'-bis(trimethylsilyl)benzamidinate]zirconium dichloride, cyclopentadienyl[N,N'-bis(trimethylsilyl)benzamidinate]zirconium dibromide, cyclopentadienyl(dimethylbenzamidinate)zirconium dichloride, and cyclopentadienyl(dimethylbenzamidinate)zirconium dibromide.

Specific examples of the transition metal compound represented by the general formula (2) are as follows.

Examples of bis(monosubstituted cyclopentadienyl) zirconocene include bis(t-butylcyclopentadienyl)zirconium dichloride, bis(t-pentylcyclopentadienyl)zirconium dichloride, bis((2-methylpentan-2-yl)cyclopentadienyl)zirconium dichloride, bis((2,4-dimethylpentan-2-yl)cyclopentadienyl)zirconium dichloride, bis((2,3-dimethylbutan-2-yl)cyclopentadienyl)zirconium dichloride, bis((2,3,3-trimethylbutan-2-yl)cyclopentadienyl)zirconium dichloride, bis((3-methylpentan-3-yl)cyclopentadienyl)zirconium dichloride, bis((3-methylhexan-3-yl)cyclopentadienyl)zirconium dichloride, bis((3,5-dimethylhexan-3-yl)cyclopentadienyl)zirconium dichloride, bis((2,2,3-trimethylpentan-3-yl)cyclopentadienyl)zirconium dichloride, bis((2,3-dimethylpentan-3-yl)cyclopentadienyl)zirconium dichloride, bis((3-ethylpentan-3-yl)cyclopentadienyl)zirconium dichloride, bis((3-ethylhexan-3-yl)cyclopentadienyl)zirconium dichloride, bis((3-ethyl-5-methylhexan-3-yl)cyclopentadienyl)zirconium dichloride, bis((3-ethyl-2-methylpentan-3-yl)cyclopentadienyl)zirconium dichloride, bis((3-ethyl-2,2-dimethylpentan-3-yl)cyclopentadienyl)zirconium dichloride, bis((1-methylcyclohexyl)cyclopentadienyl)zirconium dichloride, bis((1-ethylcyclohexyl)cyclopentadienyl)zirconium dichloride, bis((1-isopropylcyclohexyl)cyclopentadienyl)zirconium dichloride, bis((1-t-butylcyclohexyl)cyclopentadienyl)zirconium dichloride, bis((1-(2,3-dimethylbutan-2-yl)cyclohexyl)cyclopentadienyl)zirconium dichloride, bis((1-methylcyclopentyl)cyclopentadienyl)zirconium dichloride, bis((1-ethylcyclopentyl)cyclopentadienyl)zirconium dichloride, bis((1-isopropylcyclopentyl)cyclopentadienyl)zirconium dichloride, bis((1-t-butylcyclopentyl)cyclopentadienyl)zirconium dichloride, bis((1-(2,3-dimethylbutan-2-yl)cyclopentyl)cyclopentadienyl)zirconium dichloride, bis((2-phenylpropan-2-yl)cyclopentadienyl)zirconium dichloride, bis((2-phenylbutan-2-yl)cyclopentadienyl)zirconium dichloride, bis((3-phenylpentan-3-yl)cyclopentadienyl)zirconium dichloride, bis((2-(2-indenyl)propan-2-yl)cyclopentadienyl)zirconium dichloride, bis((2-(2-indenyl)butan-2-yl)cyclopentadienyl)zirconium dichloride, bis((3-(2-indenyl)pentan-3-yl)cyclopentadienyl)zirconium dichloride, bis((2-(2-indenyl)propan-2-yl)cyclopentadienyl)zirconium dichloride, bis((2-indenylbutan-2-yl)cyclopentadienyl)zirconium dichloride, bis((3-indenylpentan-3-yl)cyclopentadienyl)zirconium dichloride, bis((1,1-diphenylethyl)cyclopentadienyl)zirconium dichloride, bis((1,1-diphenylpropyl)cyclopentadienyl)zirconium dichloride, and bis((1,1-diphenylbutyl)cyclopentadienyl)zirconium dichloride.

Examples of bis(disubstituted cyclopentadienyl) zirconocene include bis(1-methyl-3-t-butylcyclopentadienyl)zirconium dichloride, bis(1-methyl-3-t-pentylcyclopentadienyl)zirconium dichloride, bis(1-methyl-3-(2-methylpentan-2-yl)cyclopentadienyl)zirconium dichloride, bis(1-methyl-3-(2,4-dimethylpentan-2-yl)cyclopentadienyl)zirconium dichloride, bis(1-methyl-3-(2,3-dimethylbutan-2-yl)cyclopentadienyl)zirconium dichloride, bis(1-methyl-3-(2,3,3-trimethylbutan-2-yl)cyclopentadienyl)zirconium dichloride, bis(1-methyl-3-(3-methylpentan-3-yl)cyclopentadienyl)zirconium dichloride, bis(1-methyl-3-(3-methylhexan-3-yl)cyclopentadienyl)zirconium dichloride, bis(1-methyl-3-(3,5-dimethylhexan-3-yl)cyclopentadienyl)zirconium dichloride, bis(1-methyl-3-(3,4-dimethylpentan-3-yl)cyclopentadienyl)zirconium dichloride, bis(1-methyl-3-(3,4,4-trimethylpentan-3-yl)cyclopentadienyl)zirconium dichloride, bis((1-methyl-3-ethylpentan-3-yl)cyclopentadienyl)zirconium dichloride, bis((1-methyl-3-ethylhexan-3-yl)cyclopentadienyl)zirconium dichloride, bis((1-methyl-(3-ethyl-5-methyl)hexan-3-yl)cyclopentadienyl)zirconium dichloride, bis((1-methyl-(3-ethyl-4-methyl)pentan-3-yl)cyclopentadienyl)zirconium dichloride, bis((1-methyl-(3-ethyl-4,4-dimethyl)pentan-3-yl)cyclopentadienyl)zirconium dichloride, bis(1-methyl-3-(1-methylcyclohexyl)cyclopentadienyl)zirconium dichloride, bis(1-methyl-3-(1-ethylcyclohexyl)cyclopentadienyl)zirconium dichloride, bis(1-methyl-3-(1-isopropylcyclohexyl)cyclopentadienyl)zirconium dichloride, bis(1-methyl-3-(1-t-butylcyclohexyl)cyclopentadienyl)zirconium dichloride, bis((1-methyl-3-(2,3-dimethylbutan-2-yl)cyclohexyl)cyclopentadienyl)zirconium dichloride, bis(1-methyl-3-(1-methylcyclopentyl)cyclopentadienyl)zirconium dichloride, bis(1-methyl-3-(1-ethylcyclopentyl)cyclopentadienyl)zirconium dichloride, bis(1-methyl-3-(1-isopropylcyclopentyl)cyclopentadienyl)zirconium dichloride, bis((1-methyl-3-(1-t-butylcyclopentyl)cyclopentadienyl)zirconium dichloride, and bis((1-methyl-3-(2,3-dimethylbutan-2-yl)cyclopentyl)cyclopentadienyl)zirconium dichloride.

Examples of bis(trisubstituted cyclopentadienyl) zirconocene include bis(1,2-dimethyl-3-t-butylcyclopentadienyl)zirconium dichloride, bis(1,2-dimethyl-3-t-pentylcyclopentadienyl)zirconium dichloride, bis(1,2-dimethyl-3-(2-methylpentan-2-yl)cyclopentadienyl)zirconium dichloride, bis(1,2-dimethyl-3-(2,4-dimethylpentan-2-yl)cyclopentadienyl)zirconium dichloride, bis(1,2-dimethyl-3-(2,3-dimethylbutan-2-yl)cyclopentadienyl)zirconium dichloride, bis(1,2-dimethyl-3-(2,3,4-trimethylbutan-2-yl)cyclopentadienyl)zirconium dichloride, bis(1,2-dimethyl-3-(3-methylpentan-3-yl)cyclopentadienyl)zirconium dichloride, bis(1,2-dimethyl-3-(3-methylhexan-3-yl)cyclopentadienyl)zirconium dichloride, bis(1,2-dimethyl-3-(2-methylpentan-2-yl)cyclopentadienyl)zirconium dichloride, bis(1,2-dimethyl-3-(2,4-dimethylpentan-2-yl)cyclopentadienyl)zirconium dichloride, bis(1,2-dimethyl-3-(2,3-dimethylbutan-2-yl)cyclopentadienyl)zirconium dichloride, bis(1,2-dimethyl-3-(2,3,4-trimethylbutan-2-yl)cyclopentadienyl)zirconium dichloride, bis(1,2-dimethyl-3-(3-ethylpentan-3-yl)cyclopentadienyl)zirconium dichloride, bis(1,2-dimethyl-3-(3-ethylhexan-3-yl)cyclopentadienyl)zirconium dichloride, bis(1,2-dimethyl-3-(3-ethyl-5-methylhexan-3-yl)cyclopentadienyl)zirconium dichloride, bis(1,2-dimethyl-3-(3-ethyl-4-methylpentan-3-yl)cyclopentadienyl)zirconium dichloride, bis(1,2-dimethyl-3-(3-ethyl-4,4-dimethylpentan-3-yl)cyclopentadienyl)zirconium dichloride, bis(1,3-dimethyl-3-(1-methylcyclohexyl)cyclopentadienyl)zirconium dichloride, bis(1,3-dimethyl-3-(1-ethylcyclohexyl)cyclopentadienyl)zirconium dichloride, bis(1,3-dimethyl-3-(1-isopropylcyclohexyl)cyclopentadienyl)zirconium dichloride, bis(1,3-dimethyl-3-(1-t-butylcyclohexyl)cyclopentadienyl)zirconium dichloride, bis(1,3-dimethyl-3-(2,3-dimethylbutan-2-yl)cyclohexyl)cyclopentadienyl)zirconium dichloride, bis(1,3-dimethyl-3-(1-methylcyclopentyl)cyclopentadienyl)zirconium dichloride, bis(1,3-dimethyl-3-(1-ethylcyclopentyl)cyclopentadienyl)zirconium dichloride, bis(1,3-dimethyl-3-(1-isopropylcyclopentyl)cyclopentadienyl)zirconium dichloride, bis(1,3-dimethyl-3-(1-t-butylcyclopentyl)cyclopentadienyl)zirconium dichloride, and bis(1,3-dimethyl-3-(2,3-dimethylbutan-2-yl)cyclopentyl)cyclopentadienyl)zirconium dichloride.

Examples of the aluminum compound (ii) that constitutes the catalyst include an organoaluminum oxo compound and an organoaluminum compound. The organoaluminum compound is preferably used in combination with an organoboron compound. That is, preferably, the metallocene catalyst further contains the organoaluminum oxo compound or the organoaluminum compound and the organoboron compound.

Examples of the organoaluminum oxo compound include a linear aluminoxane represented by a general formula (4) and a cyclic aluminoxane represented by a general formula (5): 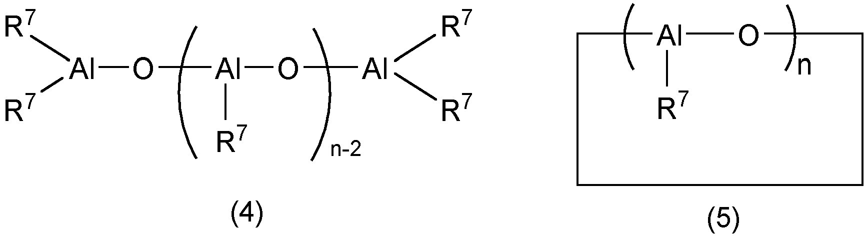 wherein R⁷'s each independently represent a hydrocarbon group such as an alkyl group, alkenyl group, aryl group, or arylalkyl group having 1 to 20 carbon atoms, and preferably 1 to 12 carbon atoms, or a halogen atom, and may be the same as or different from each other, and n represents a polymerization degree and represents usually an integer of 3 to 50, and preferably 7 to 40.

In particular, methylaluminoxane (MAO) wherein R⁷'s are a methyl group is preferred.

A solution in which methylaluminoxane (MAO) is dissolved in a solvent is preferably used. Examples of the used solvent include aromatic hydrocarbons such as benzene, toluene, xylene, and ethylbenzene. Toluene is preferred.

Examples of the organoaluminum compound include trimethylaluminum, triethylaluminum, triisopropylaluminum, triisobutylaluminum (TIBA), dimethylaluminum chloride, diethylaluminum chloride, methylaluminum dichloride, ethylaluminum dichloride, dimethylaluminum fluoride, diisobutylaluminum hydride, diethylaluminum hydride, and ethylaluminum sesquichloride. Triisobutylaluminum (TIBA) is preferred.

Examples of the organoboron compound include triphenylboron, tris(pentafluorophenyl)boron, tris[3,5-bis(trifluoromethyl)phenyl]boron, tris[(4-fluoromethyl)phenyl]boron, trimethylboron, triethylboron, tri-n-butylboron, tris(fluoromethyl)boron, tris(pentafluoroethyl)boron, tris(nonafluorobutyl)boron, tris(2,4,6-trifluorophenyl)boron, tris(3,5-difluoro)boron, tris[3,5-bis(trifluoromethyl)phenyl]boron, bis(pentafluorophenyl)fluoroboron, diphenylfluoroboron, bis(pentafluorophenyl)chloroboron, dimethylfluoroboron, diethylfluoroboron, di-n-butylfluoroboron, pentafluorophenyldifluoroboron, phenyldifluoroboron, pentafluorophenyldichloroboron, methylfluoroboron, ethylfluoroboron, and n-butyldifluoroboron.

In addition, examples thereof include triethylammonium tetraphenylborate, tri-n-butylammonium tetraphenylborate, trimethylammonium tetraphenylborate, tetraethylammonium tetraphenylborate, methyl(tri-n-butyl)ammonium tetraphenylborate, benzyl(tri-n-butyl)ammonium tetraphenylborate, dimethyldiphenylammonium tetraphenylborate, triphenyl(methyl)ammonium tetraphenylborate, trimethylanilinium tetraphenylborate, methylpyridinium tetraphenylborate, benzylpyridinium tetraphenylborate, methyl(2-cyanopyridinium) tetraphenylborate, triethylammonium tetrakis(pentafluorophenyl)borate, tri-n-butylammonium tetrakis(pentafluorophenyl)borate, triphenylammonium tetrakis(pentafluorophenyl)borate, tetra-n-butylammonium tetrakis(pentafluorophenyl)borate, tetraethylammonium tetrakis(pentafluorophenyl)borate, benzyl(tri-n-butyl)ammonium tetrakis(pentafluorophenyl)borate, methyldiphenylammonium tetrakis(pentafluorophenyl)borate, triphenyl(methyl)ammonium tetrakis(pentafluorophenyl)borate, methylanilinium tetrakis(pentafluorophenyl)borate, dimethylanilinium tetrakis(pentafluorophenyl)borate, trimethylanilinium tetrakis(pentafluorophenyl)borate, methylpyridinium tetrakis(pentafluorophenyl)borate, benzylpyridinium tetrakis(pentafluorophenyl)borate, methyl(2-cyanopyridinium) tetrakis(pentafluorophenyl)borate, benzyl(2-cyanopyridinium) tetrakis(pentafluorophenyl)borate, methyl(4-cyanopyridinium) tetrakis(pentafluorophenyl)borate, triphenylphosphonium tetrakis(pentafluorophenyl)borate, dimethylanilinium tetrakis[bis(3,5-ditrifluoromethyl)phenyl]borate, ferrocenium tetraphenylborate, silver tetraphenylborate, trityl tetraphenylborate, tetraphenylporphyrinmanganese tetraphenylborate, ferrocenium tetrakis(pentafluorophenyl)borate, (1,1'-dimethylferrocenium) tetrakis(pentafluorophenyl)borate, decamethylferrocenium tetrakis(pentafluorophenyl)borate, silver tetrakis(pentafluorophenyl)borate, trityl tetrakis(pentafluorophenyl)borate, lithium tetrakis(pentafluorophenyl)borate, sodium tetrakis(pentafluorophenyl)borate, tetraphenylporphyrinmanganese tetrakis(pentafluorophenyl)borate, silver tetrafluoroborate, silver hexafluorophosphate, silver hexafluoroarsenate, silver perchlorate, silver trifluoroacetate, and silver trifluoromethanesulfonate.

In the metallocene catalyst, the use ratio by mole (organoaluminum oxo compound/transition metal compound) of the organoaluminum oxo compound to the transition metal compound (metallocene complex) is preferably 20:1 to 400:1, and more preferably 50:1 to 200:1.

When the organoaluminum compound is used in combination with the organoboron compound, the use ratio by mole (organoaluminum compound/transition metal compound) of the organoaluminum compound to the transition metal compound (metallocene complex) is preferably 1:1 to 10,000:1, more preferably 5:1 to 2,000:1, and even more preferably 10:1 to 1,000:1. The use ratio by mole (organoboron compound/transition metal compound) of the organoboron compound to the transition metal compound (metallocene complex) is preferably 1:10 to 100:1, and more preferably 1:2 to 10:1.

When the use ratio of the aluminum compound falls within the above-described range, the polymerization degree can be easily controlled, and the selectivity can be enhanced.

### <α-Olefin>

An α-olefin refers to an alkene having a carbon-carbon double bond in the α position (at the terminal).

The α-olefin used in the oligomerization step A1 is preferably an α-olefin having 6 to 12 carbon atoms, and more preferably an α-olefin having 8 to 10 carbon atoms.

Furthermore, it is preferably a linear α-olefin represented by the following general formula, more preferably a linear α-olefin having 6 to 12 carbon atoms, and even more preferably a linear α-olefin having 8 to 10 carbon atoms.

H₂C=CH-(CH₂)ₙ-CH₃

wherein n represents an integer of 3 to 9.

Specific examples of the α-olefin include 1-octene, 1-decene, 1-dodecene, 1-tridecene, 1-tetradecene, 1-pentadecene, 1-hexadecene, 1-heptadecene, and 1-octadecene. 1-octene, 1-decene, 1-dodecene, and 1-tetradecene are preferred, 1-octene and 1-decene are more preferred, and 1-decene is even more preferred. That is, the α-olefin in the oligomerization step A1 is even more preferably 1-decene.

One type of the α-olefin may be used, or two or more types thereof may be used in combination.

### <Oligomerization Condition in Oligomerization Step A1>

An oligomerization condition in the oligomerization step A1 is as follows.

The oligomerization temperature is preferably 0 to 200°C, more preferably 40 to 140°C, more preferably 40 to 100°C, and even more preferably 40 to 60°C.

When the oligomerization temperature falls within the above-described range, the polymerization degree of the resulting oligomer can be easily adjusted, and the selectivity can be enhanced.

The pressure upon oligomerization is preferably an atmospheric pressure to 1 MPaG, and more preferably an atmospheric pressure to 0.2 MPaG. Herein, "MPaG" represents "MPa (gauge pressure)". The oligomerization time is preferably 0.5 to 50 hours.

In the oligomerization step A1, a solvent may be used. Examples of the solvent include aromatic hydrocarbons such as benzene, toluene, xylene, and ethylbenzene. Toluene is preferred.

The oligomerization reaction is preferably terminated by mixing a base with the reaction liquid after the oligomerization. The base that can be used is preferably at least one selected from the group consisting of an alkali metal hydroxide, ammonia, and amines, and more preferably an alkali metal hydroxide.

Examples of the alkali metal hydroxide include sodium hydroxide and potassium hydroxide. An aqueous solution of the alkali metal hydroxide is preferably mixed with the reaction liquid.

In order to decompose and remove the catalyst and the catalyst that is neutralized with the base, the reaction liquid is preferably washed with an aqueous solvent such as water.

Preferably, prior to the distillation separation step that is a subsequent step, the reaction liquid is heated and depressurized, if necessary, to remove the solvent and an unreacted α-olefin.

### [Distillation Separation Step A2]

The distillation separation step A2 is a step of distilling and separating the oligomer (a) obtained in the oligomerization step A1 to obtain the high molecular weight fraction (a1) and the low molecular weight fraction (a2). In the method for producing an α-olefin oligomer composition of the present invention, this step is performed prior to the oligomerization step B1 and the mixing step C, and of the obtained fractions, a portion or the whole of the low molecular weight fraction (a2) are subjected to the oligomerization step B1. The low molecular weight fraction (a2) that has not been used in the oligomerization step B1, and the fraction that is obtained in this step and contains the high molecular weight fraction (a1) may be subjected to the mixing step C. In particular, the high molecular weight fraction (a1) is preferably subjected to the mixing step C.

For the distillation separation step A2, a distillation condition may be determined such that each resulting fraction has an intended polymerization degree (molecular weight), but the condition is preferably as follows.

The distillation temperature is preferably 200°C to 300°C, preferably 220 to 280°C, and even more preferably 230 to 270°C. The pressure is preferably 0.1 to 15 Pa, more preferably 0.4 to 7 Pa, and even more preferably 0.6 to 4 Pa.

In this step, the condition is preferably set particularly such that the high molecular weight fraction (a1) is a fraction with more than 20 carbon atoms and the low molecular weight fraction (a2) is a fraction with 20 or less carbon atoms.

That is, preferably, in the distillation separation step A2, the resulting high molecular weight fraction (a1) is a fraction with more than 20 carbon atoms and the resulting low molecular weight fraction (a2) is a fraction with 20 or less carbon atoms.

### [Hydrogenation Step A3]

A hydrogenation step A3 is an optional step, which is a step of hydrogenating the high molecular weight fraction (a1) obtained in the distillation separation step A2 to obtain a hydrogenated oligomer (a11). The obtained hydrogenated oligomer (a11) is preferably subjected to the mixing step C described below.

When the high molecular weight fraction (a1) is hydrogenated, the resulting hydrogenated oligomer is a saturated hydrocarbon, the stability against exogenous stimulus is enhanced during use as a lubricating oil, and the lubricating oil can withstand long-term use.

A reaction condition in the hydrogenation step A3 may be a condition of a general hydrogenation reaction, and is preferably as follows.

In this hydrogenation step, the high molecular weight fraction (a1) is preferably hydrogenated in a gas phase with a hydrogenation catalyst to obtain the hydrogenated oligomer (a11).

A common gas-phase hydrogenation method can be used in the hydrogenation step. When a noble metal catalyst, such as palladium or platinum, is used as the catalyst, the reaction temperature is preferably 60 to 100°C and the hydrogen pressure is preferably 0.1 to 1 MPa. When a nickel catalyst is used, the reaction temperature is preferably 150 to 250°C and the hydrogen pressure is preferably 1 to 20 MPa. In either catalytic system, the catalytic amount is preferably 0.05 to 50 mass% of the amount of the high molecular weight fraction (a1), and the reaction time is preferably 2 to 48 hours. The hydrogenation reaction proceeds rapidly by using the above-described hydrogenation catalyst. An additional operation, for example, to raise the temperature or pressure of the reaction system, may be carried out even after noticeable hydrogen absorption ceases in order to completely perform hydrogenation of a small amount of residual starting material.

### [Oligomerization Step B1]

The oligomerization step B1 is an oligomerization step of oligomerizing an α-olefin oligomer containing the low molecular weight fraction (a2) with an acid catalyst to obtain the oligomer (b). In the distillation separation step A2, the low molecular weight fraction (a2) is obtained by distilling and separating the oligomer (a) obtained in the oligomerization step A1.

### <Acid Catalyst>

Examples of the acid catalyst used in the oligomerization step B 1 include a Friedel-Crafts catalyst, a solid acid catalyst, a Lewis acid catalyst, and a Bronsted acid catalyst. Among them, a Friedel-Crafts catalyst is preferred.

The Friedel-Crafts catalyst preferably contains an organoaluminum compound, and more preferably contains an organoaluminum compound and an organic halide.

Examples of the organoaluminum compound include trialkylaluminum, dialkylaluminum halide, and alkylaluminum dihalide. Dialkylaluminum halide is preferred.

Specific examples of the organoaluminum compound include trimethylaluminum, triethylaluminum, triisobutylaluminum, diethylaluminum chloride, ethylaluminum sesquichloride, and ethylaluminum dichloride. Among them, diethylaluminum chloride is preferred.

Examples of the organic halide include an alkyl halide and an allyl halide. An alkyl halide is preferred.

Specific examples of the alkyl halide include t-butyl chloride, sec-butyl chloride, cyclohexyl chloride, and 2,5-dimethyl-2-chlorohexane. t-butyl chloride is preferred.

The molar ratio of the organoaluminum compound to the organic halide (organoaluminum compound/organic halide) in this step is preferably 1/10 to 1/0.5, more preferably 1/5 to 1/1, and even more preferably 1/4 to 1/2. When the ratio is 1/10 or more, the halogen content of the resulting oligomer can be reduced, leading to easy removal of halogen. When the ratio is 1/0.5 or less, the reaction can be performed with high reproducibility.

The concentration of the Friedel-Crafts catalyst used in this step, as determined in terms of the molar amount of aluminum per volume of the substrate (olefin) at 25°C, is preferably 0.5 to 50 mmol/L, more preferably 0.6 to 20 mmol/L, even more preferably 0.8 to 10 mmol/L, and still more preferably 1 to 5 mmol/L. When the concentration of the catalyst is 0.5 mmol/L or more, the reaction can be performed with high reproducibility. When the concentration of the catalyst is 50 mmol/L or less, the halogen content of the resulting oligomer can be reduced, leading to easy removal of halogen.

### <α-Olefin Oligomer>

The α-olefin oligomer used in the oligomerization step B 1 contains the low molecular weight fraction (a2).

The low molecular weight fraction (a2) is the low molecular weight fraction obtained in the distillation separation step A2.

Since the low molecular weight fraction (a2) is obtained by oligomerizing the α-olefin with the metallocene catalyst in the oligomerization step A1 and then distilling and separating the resulting oligomer, the low molecular weight fraction (a2) contains as a main component the α-olefin oligomer preferably used in this step. Since the low molecular weight fraction (a2) further contains a dimer obtained by dimerizing an α-olefin and a vinylidene olefin having a vinylidene structure, it is preferably used as a starting material in this step, and the finally obtained α-olefin oligomer composition tends to have an intended polymerization degree and is excellent in performance during use as a lubricating oil.

When the low molecular weight fraction (a2) is used for the starting material in this step, a low molecular weight component that is not appropriate for a lubricating oil, of the oligomer (a) obtained in the oligomerization step A1, can be utilized.

In the α-olefin oligomer used in the oligomerization step B1, an α-olefin oligomer having a relatively low polymerization degree may be used as the low molecular weight fraction (a2). In particular, a dimer obtained by dimerizing an α-olefin is preferred, and a vinylidene olefin having a vinylidene structure is more preferred.

Preferably, the vinylidene olefin is at least one selected from compounds represented by the following general formula (6): 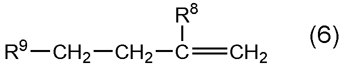 wherein R⁸ and R⁹ each independently represent a hydrogen atom or a linear or branched alkyl group having 1 to 16 carbon atoms.

In the general formula (6), R⁸ and R⁹ are each independently a hydrogen atom or a linear or branched alkyl group having 1 to 16 carbon atoms, and are preferably a linear alkyl group having 8 to 16 carbon atoms in the present invention. Examples of the liner alkyl group having 8 to 16 carbon atoms include a n-octyl group, a n-nonyl group, a n-decyl group, a n-undecyl group, a n-dodecyl group, a n-tridecyl group, a n-tetradecyl group, a n-pentadecyl group, and a n-hexadecyl group.

### <Oligomerization Condition in Oligomerization Step B 1>

Preferably, prior to initiating the reaction in this step, a treatment is performed to remove moisture, an oxidation product, etc., from the starting material. The treatment may be performed, for example, by a method which involves putting an adsorbent in the starting material for adsorption removal of moisture, etc., or a method which involves bubbling an inert gas or a dried gas into the olefin to remove moisture, etc., with a gas flow. Such methods are preferably used in combination.

The adsorbent is preferably activated alumina or a molecular sieve.

Nitrogen gas is preferably used as a bubbling gas.

The oligomerization reaction is allowed to proceed through contact between the catalyst and the olefin oligomer.

The reaction temperature upon the oligomerization reaction is preferably 0 to 100°C, more preferably 25 to 90°C, and even more preferably 30 to 80°C. When the reaction temperature is 0°C or more, the time until the initiation of the reaction can be reduced and, in addition, the reaction can be performed with high reproducibility. When the reaction temperature is 100°C or less, the intended oligomer can be obtained at a high yield without deactivation of the catalyst and a side reaction.

The reaction is an exothermic reaction; therefore, the reaction temperature increases during the reaction. The maximum reaction temperature is preferably adjusted to be within the above range. The end point of the reaction can be determined by detection of no generation of heat.

### [Isomerization Step B2]

An isomerization step B2 is an optional step, which is a step of isomerizing the oligomer (b) obtained in the oligomerization step B 1 to obtain an isomer. Preferably, this step is followed by a hydrogenation step B3 of hydrogenating the resulting isomer to obtain a hydrogenated isomer, and the resulting hydrogenated isomer is subjected to the mixing step C. That is, preferably, the method for producing an α-olefin oligomer composition of the present invention further includes the isomerization step B2 of isomerizing the oligomer (b) to obtain an isomer and the hydrogenation step B3 of hydrogenating the isomer to obtain a hydrogenated isomer, and the resulting hydrogenated isomer is subjected to the mixing step C.

### <Acid Catalyst>

The isomerization step B2 is preferably isomerization in the presence of an acid catalyst.

Examples of the acid catalyst include a Friedel-Crafts catalyst, a solid acid catalyst, a Lewis acid catalyst, and a Bronsted acid catalyst. Among them, a Friedel-Crafts catalyst is more preferred. Thus, the isomerization step B2 is more preferably isomerization in the presence of a Friedel-Crafts catalyst.

The Friedel-Crafts catalyst preferably contains an organoaluminum compound, and more preferably contains an organoaluminum compound and an organic halide.

### Examples of the organoaluminum compound include trialkylaluminum, dialkylaluminum halide, and alkylaluminum dihalide. Dialkylaluminum halide is preferred

Specific examples of the organoaluminum compound include trimethylaluminum, triethylaluminum, triisobutylaluminum, diethylaluminum chloride, ethylaluminum sesquichloride, and ethylaluminum dichloride. Among them, diethylaluminum chloride is preferred.

Examples of the organic halide include an alkyl halide and an allyl halide. An alkyl halide is preferred.

Specific examples of the alkyl halide include t-butyl chloride, sec-butyl chloride, cyclohexyl chloride, and 2,5-dimethyl-2-cyclohexane. t-butyl chloride is preferred.

The molar ratio of the organoaluminum compound to the organic halide (organoaluminum compound/organic halide) in this step is preferably 1/10 to 1/0.5, more preferably 1/5 to 1/1, and even more preferably 1/4 to 1/2. When the ratio is 1/10 or more, the halogen content of the resulting isomer can be reduced, leading to easy removal of halogen. When the ratio is 1/0.5 or less, the reaction can be performed with high reproducibility.

The concentration of the Friedel-Crafts catalyst used in this step, as determined in terms of the molar amount of aluminum per volume of the substrate (oligomer) at 25°C, is preferably 0.5 to 100 mmol/L, more preferably 1 to 40 mmol/L, even more preferably 1.5 to 20 mmol/L, and still more preferably 2 to 10 mmol/L. When the concentration of the catalyst is 0.5 mmol/L or more, the reaction can be performed with high reproducibility. When the concentration of the catalyst is 100 mmol/L or less, the halogen content of the resulting isomer can be reduced, leading to easy removal of halogen. In the case wherein a Friedel-Crafts catalyst is used in the step B 1, and the reaction mixture obtained in the step B 1 is used as it is in the step B2, the above-described concentration of the Friedel-Crafts catalyst refers to the concentration of the total amount of the catalyst, including the catalyst used in the step B 1.

### <Reaction Condition of Isomerization Step B2>

The reaction temperature upon the isomerization reaction is preferably 120 to 200°C, more preferably 130 to 190°C, and even more preferably 140 to 180°C. When the reaction temperature is 120°C or more, the isomerization proceeds efficiently in a short time. When the reaction temperature is 200°C or less, the intended isomer can be obtained at a high yield without a degradation reaction.

It appears that the oligomer (b) (olefin oligomer) obtained in the above-described step is isomerized into a more stable structure by performing the isomerization at a predetermined temperature using the above-described catalyst. Though the reason for this is not fully elucidated, this is considered to be because a quaternary carbon moiety is reduced by isomerization.

The reaction time upon the isomerization reaction is preferably 1 to 240 minutes, more preferably 2 to 180 minutes, even more preferably 3 to 160 minutes, and still more preferably 5 to 140 minutes. When the reaction time is 240 minutes or less, the intended isomer can be obtained at a high yield without a side reaction such as an oligomerization reaction.

The isomerization reaction is preferably terminated by adding an alkali, such as sodium hydroxide, to the reaction system.

After the termination of the reaction, the reaction mixture is preferably washed with water to remove the catalyst, a salt derived from the catalyst, etc. The washing with water is preferably performed in such a manner that the reaction mixture, which has once been made alkaline, is brought to neutral, and that the pH of water used in the washing becomes 9 or less.

### [Hydrogenation Step B3]

The hydrogenation step B3 is an optional step, which is a step of hydrogenating the isomer to obtain a hydrogenated isomer. Preferably, the hydrogenated isomer obtained in this step is subjected to the mixing step C. More preferably, the method for producing an α-olefin oligomer composition of the present invention further includes, after this step, a distillation separation step B4 of distilling and separating the resulting hydrogenated isomer to obtain a high molecular weight fraction (b11) and a low molecular weight fraction (b21), and the high molecular weight fraction (b11) is subjected to the mixing step C.

Preferably, in the hydrogenation step, the isomer is hydrogenated in a gas phase with a hydrogenation catalyst, to obtain the hydrogenated isomer.

A common gas-phase hydrogenation method can be used in the hydrogenation step. When a noble metal catalyst, such as palladium or platinum, is used as the catalyst, the reaction is preferably performed at a reaction temperature of 60 to 100°C and a hydrogen pressure of 0.1 to 1 MPa. When a nickel catalyst is used, the reaction is preferably performed at a reaction temperature of 150 to 250°C and a hydrogen pressure of 1 to 20 MPa. In either catalytic system, the catalytic amount is generally 0.05 to 50 mass% of the amount of the isomer, and the hydrogenation reaction is completed in 2 to 48 hours. The hydrogenation reaction proceeds rapidly with the above-described hydrogenation catalyst. An additional operation, for example, to raise the temperature or pressure of the reaction system, may be carried out even after noticeable hydrogen absorption ceases in order to completely perform hydrogenation of a small amount of residual unsaturated hydrocarbon compound.

### [Distillation Separation Step B4]

The distillation separation step B4 is an optional step, which is a step of distilling and separating the hydrogenated isomer obtained in the hydrogenation step B3 to obtain the high molecular weight fraction (b11) and the low molecular weight fraction (b21).

In the method for producing an α-olefin oligomer composition of the present invention, this step may be performed prior to the mixing step C, and the resulting fraction may be subjected to the mixing step C. In particular, the high molecular weight fraction (b11) is preferably subjected to the mixing step C.

For the distillation separation step B4, a distillation condition may be determined such that each resulting fraction has an intended polymerization degree (molecular weight), but the condition is preferably as follows.

The distillation temperature is preferably 200°C to 300°C, preferably 220 to 280°C, and even more preferably 230 to 270°C. The pressure is preferably 0.1 to 15 Pa, more preferably 0.4 to 7 Pa, and even more preferably 0.6 to 4 Pa.

In this step, the condition is preferably set particularly such that the high molecular weight fraction (b 11) is a fraction with more than 20 carbon atoms and the low molecular weight fraction (b21) is a fraction with 20 or less carbon atoms.

That is, preferably, in the distillation separation step B4, the resulting high molecular weight fraction (b11) is a fraction with more than 20 carbon atoms and the resulting low molecular weight fraction (b21) is a fraction with 20 or less carbon atoms.

### [Mixing Step C]

The mixing step C is a step of mixing a portion of the oligomer (a) or a hydrogenated product thereof and a portion or the whole of the oligomer (b), the isomer thereof, or the hydrogenated isomer thereof to obtain the α-olefin oligomer composition.

That is, in the mixing step C, a mixture is obtained by mixing the oligomer (α-olefin oligomer) obtained in the oligomerization step A1 that is a first step and the oligomer (α-olefin oligomer) obtained in the oligomerization step B1 that is a first step.

The "one portion of the oligomer (a) or the hydrogenated product thereof" in this step is preferably at least one including the oligomer (a) obtained in the oligomerization step A1, the high molecular weight fraction (a1) obtained in the distillation separation step A2, and the hydrogenated oligomer (a11) obtained in the hydrogenation step A3. The "one portion or the whole of the oligomer (b), the isomer thereof, or the hydrogenated isomer thereof" in this step is preferably at least one including the oligomer (b) obtained in the oligomerization step B1, the isomer obtained in the isomerization step B2, the hydrogenated isomer obtained in the hydrogenation step B3, and the high molecular weight fraction (b11) obtained in the distillation separation step B4.

The following shows suitable combinations of:
(1) the oligomer (a) obtained in the oligomerization step A1 and the oligomer (b) obtained in the oligomerization step B1;
(2) the oligomer (a) obtained in the oligomerization step A1 and the isomer obtained in the isomerization step B2;
(3) the oligomer (a) obtained in the oligomerization step A1 and the hydrogenated isomer obtained in the hydrogenation step B3;
(4) the oligomer (a) obtained in the oligomerization step A1 and the high molecular weight fraction (b11) obtained in the distillation separation step B4;
(5) the high molecular weight fraction (a1) obtained in the distillation separation step A2 and the oligomer (b) obtained in the oligomerization step B1;
(6) the high molecular weight fraction (a1) obtained in the distillation separation step A2 and the isomer obtained in the isomerization step B2;
(7) the high molecular weight fraction (a1) obtained in the distillation separation step A2 and the hydrogenated isomer obtained in the hydrogenation step B3;
(8) the high molecular weight fraction (a1) obtained in the distillation separation step A2 and the high molecular weight fraction (b11) obtained in the distillation separation step B4;
(9) the hydrogenated oligomer (a11) obtained in the hydrogenation step A3 and the oligomer (b) obtained in the oligomerization step B1;
(10) the hydrogenated oligomer (a11) obtained in the hydrogenation step A3 and the isomer obtained in the isomerization step B2;
(11) the hydrogenated oligomer (a11) obtained in the hydrogenation step A3 and the hydrogenated isomer obtained in the hydrogenation step B3; and
(12) the hydrogenated oligomer (a11) obtained in the hydrogenation step A3 and the high molecular weight fraction (b11) obtained in the distillation separation step B4.

Among them, (11) the combination of the hydrogenated oligomer (a11) obtained in the hydrogenation step A3 and the hydrogenated isomer obtained in the hydrogenation step B3 or (12) the combination of the hydrogenated oligomer (a11) obtained in the hydrogenation step A3 and the high molecular weight fraction (b11) obtained in the distillation separation step B4 is preferred, and (12) the hydrogenated oligomer (a11) obtained in the hydrogenation step A3 and the high molecular weight fraction (b11) obtained in the distillation separation step B4 is more preferred.

When the hydrogenated oligomer (a11) and the hydrogenated isomer or the high molecular weight fraction (b11) are subjected to the mixing step C, favorable selectivity is achieved, and the ratio of the resulting α-olefin oligomer composition having an intended polymerization degree is high. Therefore, an α-olefin oligomer composition having excellent properties can be obtained particularly as a lubricating oil. Since the resulting α-olefin oligomer composition is a saturated hydrocarbon and contains a stabilized isomer, the α-olefin oligomer composition can be a lubricating oil that is not deteriorated during long-term use without degeneration due to exogenous stimulus.

### [Distillation Separation Step D]

A distillation separation step D is a step of separating the mixture obtained in the mixing step C into a plurality of fractions by distillation. The method for producing an α-olefin oligomer composition preferably further includes the distillation separation step D of separating the mixture obtained in the mixing step C into a plurality of fractions by distillation.

For the distillation separation step D, a distillation condition may be determined such that each resulting fraction has an intended polymerization degree (molecular weight), but the condition is preferably as follows.

The distillation temperature is preferably 200°C to 300°C, preferably 220 to 280°C, and even more preferably 230 to 270°C. The pressure is preferably 0.1 to 15 Pa, more preferably 0.4 to 7 Pa, and even more preferably 0.6 to 4 Pa.

The distillation separation step D is preferably short path distillation. When short path distillation is performed, the resulting α-olefin oligomer composition is less deteriorated, and a high-quality lubricating oil is obtained.

### Examples

The following examples illustrate the present invention in greater detail and are not intended to limit the scope of the present invention.

A method for analyzing an α-olefin oligomer as a starting material, an intermediate product, and an α-olefin oligomer composition as a product in each of Examples and Comparative Examples is as follows.

### (1) Composition (Distribution of Carbon Atoms)

Using a solution of 0.1 g of a sample (an α-olefin oligomer as a starting material, an intermediate product, and an α-olefin oligomer composition as a product) in 25 mL of toluene, the composition of the composition was determined by a gas chromatograph (Agilent 6890N, manufactured by Agilent Technologies, Inc.) under the following condition. Components corresponding to varying carbon atoms were calculated from the resulting chromatogram, to determine the composition (distribution of carbon atoms).

### (Measurement Condition)

Column: TC-1 (30 mm × 0.25 mm, film thickness 0.25 µm, manufactured by GL Sciences Inc.)
Column oven temperature: increased from 100°C (hold time 0 min) to 320°C (hold time 10 min) at a rate of 10°C/min
Injection port temperature: 300°C
Detector temperature: 320°C
Detector: FID
Carrier gas: He
Flow rate: 1 mL/min
Injection volume: 1.0 µL
Split: 1/50

### Production Example 1 (Production of Hydrogenated Oligomer (a11) by Oligomerization with Metallocene Catalyst and Hydrogenation)

### (Oligomerization Step A1)

600 mL of 1-decene degassed and dehydrated by nitrogen bubbling was placed into a 1-L stainless steel autoclave purged with nitrogen, and then heated to 65°C. Subsequently, a toluene solution of methylaluminoxane having an adjusted methylaluminoxane concentration of 4,000 µmol/L was added, and a toluene solution of bis(t-butylcyclopentadienyl)zirconium dichloride having an adjusted zirconium concentration of 64 µmol/L was further added. Next, the pressure was increased to 3 kPa by hydrogen, an oligomerization reaction was caused at 40°C for 6 hours, and 500 mL of 1% aqueous sodium hydroxide solution was added to terminate the reaction. The resulting reaction mixture was washed twice with 100 mL of deionized water, to decompose and remove a catalyst component. The solvent and unreacted starting materials were removed under a condition of 150°C and 1.3 Pa, to obtain an oligomer (a).

### (Distillation Separation Step A2)

The oligomer (a) was subjected to distillation and separation at 1.33 Pa and 200 to 270°C using a single distillation device, to obtain a high molecular weight fraction (a1) containing 30 mass% or more of an oligomer having 30 or more carbon atoms and a low molecular weight fraction (a2) containing 95 mass% or more of an oligomer having 20 carbon atoms.

### (Hydrogenation Step A3)

The high molecular weight fraction (a1) was placed into a 5-L stainless steel autoclave purged with nitrogen, a palladium/alumina catalyst (5% Pd supported catalyst) was added in an amount of 1 mass% relative to the amount of the high molecular weight fraction (a1), hydrogen was continuously supplied at 0.8 MPa, and a reaction was caused at 85°C for 5 hours with stirring. Subsequently, the catalyst was removed by filtration, to obtain a hydrogenated oligomer (a11).

### Production Example 2 (Production of Hydrogenated High Molecular Weight Isomer (b11) by Oligomerization with Acid Catalyst, Isomerization, and Hydrogenation)

### (Oligomerization Step B1)

To the low molecular weight fraction (a2) (mainly containing a dimer of 1-decene; hereinafter referred to as α-olefin oligomer in this step) obtained in the distillation separation step A2, activated alumina (NKHO-24, manufactured by Sumitomo Chemicals Co., Ltd.) was added, and the mixture was bubbled with nitrogen to remove moisture, and the like. Thus, a dried α-olefin oligomer was obtained. A three-necked flask equipped with a three-way cock, a thermometer, and a stirring bar was purged with nitrogen, and then charged with 1,968 mL of the dried α-olefin oligomer. While stirring the dried α-olefin oligomer, the flask was heated in an oil bath to bring the temperature of the dried α-olefin oligomer to 30°C. 6.0 mmol of tert-butyl chloride and 2.0 mmol of diethylaluminum chloride (DEAC), as a catalyst, were added to the dried α-olefin oligomer. The tert-butyl chloride and the diethylaluminum chloride (DEAC) were each added in the form of a prepared solution diluted to 0.5 mol/L with the dried α-olefin oligomer. 10 minutes after the addition of the catalyst, the temperature of the reaction solution began to rise, and then the rise of temperature stopped in two minutes, indicating the termination of oligomerization.

### (Isomerization Step B2)

Next, the temperature of the reaction solution was brought to 150°C, and 6.0 mmol of tert-butyl chloride and 2.0 mmol of diethylaluminum chloride (DEAC), as a catalyst, were added to the reaction solution. The addition method was the same as that in the above-described step. A reaction was performed at 150°C for one hour, the reaction solution was cooled to 60°C, 160 mL of a 1.0 mol/L aqueous sodium hydroxide solution was added and then stirred, and the aqueous sodium hydroxide solution was removed. Subsequently, an operation of adding ion-exchange water and stirring the mixture, followed by removal of the ion-exchange water (washing with water) was performed repeatedly and terminated when the pH of ion-exchange water after washing reached 9 or less. Magnesium sulfate for drying was added to the solution to obtain an oligomer (b) (α-olefinoligomer mixture containing 50 mass% or more of oligomer having 40 carbon atoms).

### (Hydrogenation Step B3)

Next, the oligomer (b) obtained in the isomerization step B2 was transferred to an autoclave, and 5 mass % palladium-alumina was added to the oligomer, and then the autoclave was purged with nitrogen, and further purged with hydrogen. Thereafter, the temperature of the oligomer was raised, and a hydrogenation reaction was performed for 24 hours under a condition of 80°C and a hydrogen pressure of 0.8 MPa, to obtain a hydrogenated isomer.

### Example 1 (Production of α-Olefin Oligomer Composition)

### (Mixing Step C)

The hydrogenated oligomer (a11) obtained in Production Example 1 and the hydrogenated isomer obtained in the hydrogenation step B3 of Production Example 2 were mixed at a mass ratio of 1:0.45, to obtain an α-olefin oligomer composition. The composition (distribution of carbon atoms) of the obtained α-olefin oligomer composition is shown in Table 1.

### Example 2 (Production of α-Olefin Oligomer Composition)

### (Oligomerization Step A1 to Hydrogenation Step B3)

An oligomer (referred to as oligomer (a')) was obtained under a condition where the temperature under the oligomerization reaction in the oligomerization step A1 of Production Example 1 was changed from 40°C to 57°C, and then subjected to the distillation separation step A2 and the hydrogenation step A3, to obtain a low molecular weight fraction (a2') and a hydrogenated oligomer (a11'). A hydrogenated isomer was obtained in the same manner as in Production Example 2 except that the low molecular weight fraction (a2') was used instead of the low molecular weight fraction (a2) in the oligomerization step B1.

### (Mixing Step C)

The hydrogenated oligomer (a11') and the hydrogenated isomer obtained by using the low molecular weight fraction (a2') were mixed at a mass ratio of 1:0.69, to obtain an α-olefin oligomer composition. The composition (distribution of carbon atoms) of the obtained α-olefin oligomer composition is shown in Table 1.

### Comparative Example 1

The hydrogenated oligomer (a11) obtained in Production Example 1 was used as an α-olefin oligomer composition in Comparative Example 1. The composition (distribution of carbon atoms) of the obtained α-olefin oligomer composition is shown in Table 1.

### Comparative Example 2

The hydrogenated oligomer (a11') was obtained in the same manner as in Production Example 1 (Production of the hydrogenated oligomer (a11)) except that the temperature under the oligomerization reaction in the oligomerization step A1 of Production Example 1 was changed from 40°C to 57°C, and then used as an α-olefin oligomer composition in Comparative Example 2. The composition (distribution of carbon atoms) of the obtained α-olefin oligomer composition is shown in Table 1.

**Table 1**

| | | Example 1 | Example 2 | Comparative Example 1 | Comparative Example 2 |
|---|---|---|---|---|---|
| 1-decene conversion rate | | 93 | 93 | 95 | 95 |
| Composition (mass%) (Unit of 1-decene) | 20 carbon atoms (dimer) | 8 | 11 | 31 | 40 |
| | 30 carbon atoms (trimer) | 23 | 28 | 23 | 28 |
| | 40 carbon atoms (tetramer) | 30 | 37 | 13 | 10 |
| | 50 carbon atoms or more (pentamer or more) | 39 | 24 | 33 | 22 |

From the assay results of the composition (distribution of carbon atoms) (gas chromatography), the conversion rate of 1-decene used in the oligomerization step A1 was calculated as a 1-decene conversion rate.

It is shown that in the α-olefin oligomer compositions obtained in Examples, the proportions of 30 carbon atoms (trimer of 1-decene) and 40 carbon atoms (tetramer of 1-decene) are high as compared with those in the compositions in Comparative Examples. A composition containing large amounts of an α-olefin oligomer having 30 carbon atoms and an α-olefin oligomer having 40 carbon atoms is useful as a lubricating oil component. This shows that according to the method of the present invention, an α-olefin oligomer in which the number of carbon atoms is useful as a lubricating oil can be selectively obtained. In Example 2, the adjustment of the temperature during oligomerization in the oligomerization step A1 can increase the proportion of a component having 20 carbon atoms in the low molecular weight fraction (a2). Therefore, the α-olefin oligomer having 30 carbon atoms and the α-olefin oligomer having 40 carbon atoms can be efficiently obtained in the oligomerization step B1.

### Examples 3 to 7 (Production of α-Olefin Oligomer Composition)

### (Distillation Separation Step D)

The α-olefin oligomer composition obtained in Example 1 was subjected to short path distillation under a condition of a pressure of 1 to 5 PaA and a temperature of 200 to 280°C, and fractionated into each fraction having an intended kinematic viscosity. Herein, "PaA" represents "Pa (absolute pressure)". As the intended kinematic viscosity of each of the fractions (each fraction), the kinematic viscosity at 40°C of a fraction 1 is 4.9 cSt, the kinematic viscosity at 40°C of a fraction 2 is 14 cSt, the kinematic viscosity at 100°C of a fraction 3 is 5.8 cSt, and the kinematic viscosity at 40°C of a fraction 4 is 45 cSt. A fraction 5 is a residue obtained by distillation under the above-described distillation condition after removal of the fractions 1 to 4. The measurement results of physical properties of the α-olefin oligomer compositions thus obtained are shown in Table 2.

### Comparative Examples 3 to 5

In Comparative Examples 3 to 5, commercially available α-olefin oligomer compositions having kinematic viscosities, like Examples 4 to 6, were subjected to the same measurement of physical properties as in Examples 4 to 6. The measurement results of physical properties are shown in Table 2. The commercially available products used herein are Durasyn 164, Durasyn 166, and Durasyn 168 manufactured by INEOS. Durasyn 164, Durasyn 166, and Durasyn 168 are all compositions containing different hydrocarbon compounds that have different molecular structures. The compounds contained in the compositions each have a randomly branched chain. The compounds are believed to be compounds in which an α-olefin is oligomerized with an acid catalyst or a boron trifluoride catalyst.

**Table 2**

| | | Example 3 | Example 4 | Comparative Example 3 | Example 5 | Comparative Example 4 | Example 6 | Comparative Example 5 | Example 7 |
|---|---|---|---|---|---|---|---|---|---|
| Evaluation item | | Fraction 1 | Fraction 2 | Durasyn 164 | Fraction 3 | Durasyn 166 | Fraction 4 | Durasyn 168 | Fraction 5 |
| Kinematic viscosity at 40°C | cSt | 4.9 | 14.3 | 17.3 | 30.1 | 30.2 | 45.3 | 47.0 | 123.9 |
| at 100°C | cSt | 1.7 | 3.5 | 3.9 | 5.8 | 6.3 | 7.8 | 7.8 | 17.0 |
| VI | - | - | 129 | 122 | 141 | 137 | 143 | 136 | 150 |
| Noack | wt% | - | 11.1 | 12.8 | 4.0 | 6.3 | 3.1 | 5.0 | - |
| CCS -35°C | mPa·s | - | 840 | 1330 | 3200 | 3700 | - | - | - |
| Pour point | °C | -25 | - | - | - | - | -68 | - | -58 |

The kinematic viscosity (at 40°C and 100°C) and the viscosity index (VI) were measured using SVM3000 manufactured by Anton Paar GmbH according to JIS K2283: 2000. Noack test was performed according to JPI-5S-41-2004, the low-temperature cranking viscosity (CCS) (at -35°C) was measured according to JIS K2010: 1993, and the pour point was measured according to JIS K2269: 1987.

Although the α-olefin oligomer compositions obtained in Examples have similar kinematic viscosities as compared with the α-olefin oligomer compositions in Comparative Examples, the evaporation loss in the Noack test is low, the low-temperature cranking viscosity (CCS viscosity) is low, and the viscosity index (VI) is high. This shows that the α-olefin oligomer composition of the present invention is excellent in performance during use as a lubricating oil since the α-olefin oligomer composition can have both low-temperature flowability and volatility that are performances contrary to each other.

## Claims

1. A method for producing an α-olefin oligomer composition comprising:
an oligomerization step A1 of oligomerizing an α-olefin with a metallocene catalyst to obtain an oligomer (a);
a distillation separation step A2 of distilling and separating the oligomer (a) to obtain a high molecular weight fraction (a1) and a low molecular weight fraction (a2);
an oligomerization step B 1 of oligomerizing an α-olefin oligomer containing the low molecular weight fraction (a2) with an acid catalyst to obtain an oligomer (b); and
a mixing step C of mixing a portion of the oligomer (a) or a hydrogenated product thereof and a portion or a whole of the oligomer (b), an isomer thereof, or a hydrogenated isomer thereof.

2. The method for producing an α-olefin oligomer composition according to claim 1, further comprising:
a distillation separation step D of separating a mixture obtained in the mixing step C into a plurality of fractions by distillation.

3. The method for producing an α-olefin oligomer composition according to claim 1 or 2, further comprising:
a hydrogenation step A3 of hydrogenating the high molecular weight fraction (a1) to obtain a hydrogenated oligomer (a11), wherein the hydrogenated oligomer (a11) is subjected to the mixing step C.

4. The method for producing an α-olefin oligomer composition according to any one of claims 1 to 3, wherein the α-olefin in the oligomerization step A1 is 1-decene.

5. The method for producing an α-olefin oligomer composition according to any one of claims 1 to 4, wherein the high molecular weight fraction (a1) is a fraction with more than 20 carbon atoms, and the low molecular weight fraction (a2) is a fraction with 20 or less carbon atoms.

6. The method for producing an α-olefin oligomer composition according to any one of claims 1 to 5, wherein the metallocene catalyst contains a transition metal compound represented by a general formula (1) described below: 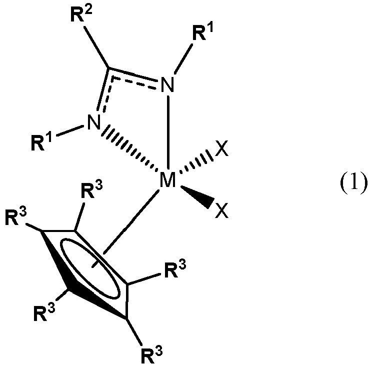 wherein M represents zirconium or hafnium, R¹'s represent an alkyl group having 1 to 10 carbon atoms or a silyl group substituted by an alkyl group having 1 to 10 carbon atoms, R² represents a phenyl group or a phenyl group substituted by an alkyl group having 1 to 10 carbon atoms, R³'s represent a hydrogen atom or a methyl group, and X's represent a halogen atom or an alkyl group having 1 to 10 carbon atoms, provided that two R¹'s may be the same as or different from each other and five R³'s may be the same as or different from each other.

7. The method for producing an α-olefin oligomer composition according to any one of claims 1 to 5, wherein the metallocene catalyst contains a transition metal compound represented by a general formula (2) described below:
{C₅H₄(AR⁴R⁵R⁶)}₂ZrCl₂ (2)
wherein R⁴'s, R⁵'s, and R⁶'s each independently represent a hydrocarbon group having 1 to 10 carbon atoms, two or more thereof may bind to each other to form a ring, and A represents a Group 14 element in the periodic table.

8. The method for producing an α-olefin oligomer composition according to any one of claims 1 to 5 or 7, wherein the metallocene catalyst contains a transition metal compound represented by a formula (3) described below:
{C₅H₄(CMe₃)}₂ZrCl₂ (3).

9. The method for producing an α-olefin oligomer composition according to any one of claims 1 to 8, wherein the metallocene catalyst further contains an organoaluminum oxo compound or an organoaluminum compound and an organoboron compound.

10. The method for producing an α-olefin oligomer composition according to any one of claims 1 to 9, further comprising:
an isomerization step B2 of isomerizing the oligomer (b) to obtain an isomer; and
a hydrogenation step B3 of hydrogenating the isomer to obtain a hydrogenated isomer, wherein the hydrogenated isomer is subjected to the mixing step C.

11. The method for producing an α-olefin oligomer composition according to claim 10, further comprising:
a distillation separation step B4 of distilling and separating the hydrogenated isomer obtained in the hydrogenation step B3 to obtain a high molecular weight fraction (b11) and a low molecular weight fraction (b21), wherein the high molecular weight fraction (b11) is subjected to the mixing step C.

12. The method for producing an α-olefin oligomer composition according to claim 11, wherein the high molecular weight fraction (b11) is a fraction with more than 20 carbon atoms, and the low molecular weight fraction (b21) is a fraction with 20 or less carbon atoms.

13. The method for producing an α-olefin oligomer composition according to any one of claims 1 to 12, wherein the acid catalyst for use in the oligomerization step B1 is a Friedel-Crafts catalyst.

14. The method for producing an α-olefin oligomer composition according to any one of claims 10 to 13, wherein the isomerization step B2 is isomerization in the presence of the Friedel-Crafts catalyst.

15. The method for producing an α-olefin oligomer composition according to claim 13 or 14, wherein the Friedel-Crafts catalyst contains an organoaluminum compound.

16. The method for producing an α-olefin oligomer composition according to any one of claims 2 to 15, wherein the distillation separation step D is short path distillation.
